Europäisches Patentamt

(19) European Patent Office

Office européen des brevets

(11) Numéro de publication: **0 121 178**
**B1**

(12) **FASCICULE DE BREVET EUROPEEN**

(45) Date de publication du fascicule du brevet:
**15.07.87**

(51) Int. Cl.⁴: **A 41 B 13/02**

(21) Numéro de dépôt: **84103086.9**

(22) Date de dépôt: **21.03.84**

(54) **Procédé de fabrication en continu de couches-culottes à jeter et couches-culottes obtenues.**

(30) Priorité: **25.03.83 FR 8304988**

(43) Date de publication de la demande:
**10.10.84 Bulletin 84/41**

(45) Mention de la délivrance du brevet:
**15.07.87 Bulletin 87/29**

(84) Etats contractants désignés:
**AT BE CH DE GB IT LI NL**

(56) Documents cités:
**EP - A - 0 027 303**
**EP - A - 0 048 010**
**FR - A - 2 415 433**
**GB - A - 2 078 811**
**GB - A - 2 118 021**
**US - A - 4 293 367**
**US - A - 4 333 782**
**US - A - 4 371 417**

(73) Titulaire: **BOUSSAC SAINT FRERES B.S.F. Société anonyme dite:, 12, rue du Vieux Faubourg, F-59800 Lille (FR)**

(72) Inventeur: **De Jonckheere, Raphael, 797 Domaine de la Vigne, F-59910 Bondues (FR)**

(74) Mandataire: **Casalonga, Axel et al, BUREAU D.A. CASALONGA OFFICE JOSSE & PETIT Morassistrasse 8, D-8000 München 5 (DE)**

ACTORUM AG

## Description

La présente invention a pour objet un procédé de fabrication de couches-culottes à jeter ainsi que les couches-culottes obtenues. On sait que les couches-culottes, qui sont utilisées généralement pour les enfants en bas âge mais aussi pour les adultes incontinents, comprennent sous une forme intégrée, à titre d'éléments principaux, une enveloppe extérieure imperméable souple jouant le rôle de culotte et un matelas absorbant fixé à l'intérieur de l'enveloppe imperméable, éventuellement recouvert d'un voile interne perméable à l'humidité. Une telle couche-culotte, que l'on appelle parfois change-complet, permet de remplacer à la fois la couche absorbante traditionnelle et la culotte imperméable qui est utilisée en combinaison avec ladite couche.

Dans le but d'améliorer l'étanchéité des couches-culottes à l'entre-jambes on a déjà prévu de disposer des éléments élastiques le long des échancrures permettant le passage des jambes pratiquées dans l'enveloppe extérieure imperméable jouant le rôle de culotte (voir par exemple les brevets américains 3 860 003 et 4 050 462).

La fixation des élastiques rectilignes par collage à l'intérieur de l'enveloppe imperméable a été faite jusqu'à présent sous tension constante, le collage étant fait au contraire de manière intermittente de sorte que seule la partie des élastiques correspondant à la zone de l'entrejambes se trouve fixée par collage sur la feuille mince se déroulant en continu destinée à former après découpage l'enveloppe extérieure imperméable de chacune des couches-culottes. La dernière étape du procédé de fabrication consiste alors à découper transversalement la bande continue constituée par la feuille destinée à former l'enveloppe extérieure imperméable, portant les matelas absorbants convenablement fixés, éventuellement un voile perméable interne, des attaches adhésives de fixation ainsi que les élastiques dont il vient d'être question. Lors de cette opération de découpe, les extrémités de ces élastiques qui n'ont pas été encollées sur la face interne de la feuille imperméable, se rétractent à l'intérieur même de la couche-culotte. On obtient donc finalement une couche-culotte dont la zone d'entrejambes comporte de chaque côté du matelas absorbant des moyens élastiques sous forte tension qui provoquent la formation de fronces ce qui a pour effet, comme cela a été constaté dans la pratique, d'améliorer l'étanchéité dans la zone de l'entrejambe. Au contraire les bords avant et arrière de la couche-culotte se trouvent démunis de telles fronces ce qui facilite la pose de la couche-culotte et sa fermeture au voisinage de la taille.

Ce procédé classique présente cependant l'inconvénient de laisser apparaître les extrémités rétractées des élastiques et de nécessiter une commande relativement complexe de l'alimentation en adhésif, laquelle alimentation doit se faire de manière intermittente.

On connaît également un procédé de fabrication en continu de couches-culottes à jeter munies de bandelettes élastiques collées en continu sur la feuille imperméable extérieure (EP-A-27 303). Dans ce procédé connu, chaque bandelette élastique est amenée à passer sur une portion de la périphérie d'un rouleau d'encollage entraîné en rotation, ce qui permet d'enduire la bandelette d'une couche d'adhésif liquide. Le rouleau d'encollage est situé en amont d'un rouleau d'alimentation entraîné en rotation à la même vitesse que le rouleau d'encollage et sur lequel la bandelette élastique encollée est enroulée sur une portion de périphérie. Après avoir quitté le rouleau d'alimentation, la bandelette élastique encollée entre en contact avec la feuille imperméable lors du passage de cette dernière sur un rouleau de guidage. Pendant une partie du fonctionnement, la vitesse d'entraînement du rouleau d'encollage et du rouleau d'alimentation est telle que la bandelette élastique encollée se déplace à la même vitesse que la feuille imperméable de sorte qu'elle se trouve fixée sans tension sur cette dernière. Pendant l'autre partie du fonctionnement, la vitesse d'entraînement du rouleau d'encollage et du rouleau d'alimentation est plus faible et telle que la bandelette élastique encollée se déplace à une vitesse inférieure à celle de la feuille imperméable de sorte qu'elle se trouve fixée sur cette dernière à l'état tendu. Etant donné cependant que l'encollage se fait en amont de la variation de tension exercée sur la bandelette élastique, il apparaît que la couche d'adhésif voit son épaisseur modifiée selon la tension de la bandelette élastique. Il en résulte une fixation dont les caractéristiques varient également entre les portions non tendues et les portions tendues de la bandelette élastique ce qui entraîne une qualité défectueuse des couches-culottes.

La présente invention a pour objet un nouveau procédé de fabrication qui permette de s'affranchir des difficultés rencontrées dans la pratique dans les procédés connus.

Le procédé selon l'invention permet la fabrication en continu de couches-culottes à jeter comprenant un coussin absorbant fixé à l'intérieur d'une enveloppe imperméable à l'humidité comportant des bords transversaux avant et arrière et une zone centrale d'entrejambe munie de découpes latérales pour permettre le passage des jambes et de moyens élastiques fixés à l'enveloppe imperméable par collage, au moins dans la zone de l'entrejambe, par contact avec la face interne de l'enveloppe imperméable entraînée en continu sur un tambour rotatif à surface lisse. Selon l'invention, lesdits moyens élastiques alimentés en continu sont encollés en continu sur toute leur longueur par application d'un adhésif liquide à haute température et l'on fait varier périodiquement la tension exercée sur lesdits moyens élastiques aux points de contact avec l'enveloppe imperméable depuis une valeur proche de zéro correspondant aux portions des moyens élastiques encollées sur les bords transversaux avant et arrière, jusqu'à une valeur maximale correspondant aux portions des moyens élastiques encollées sur la zone centrale d'entrejambe. Chaque moyen élastique passe entre deux organes d'alimentation rotatifs capables d'exercer un effet de pincement sur les moyens élastiques et entraînés à vitesse variable pour provoquer la tension variable des moyens élastiques en aval des organes d'alimentation et l'encollage des moyens élastiques est réalisé en aval des organes d'alimentation précités.

On peut utiliser pour la mise en œuvre du procédé de l'invention des rubans élastiques qui reçoivent sur l'une de leurs faces un adhésif liquide à haute température (hot melt) déposé en continu selon le procédé de l'invention. On peut également utiliser une pluralitée de brins élastiques individuels qui sont obtenus par séparation en continu à partir d'un ruban élastique multibrins. Chaque brin individuel est alors enrobé en continu sur toute sa périphérie par un adhésif liquide à haute température (hot melt).

Dans tous les cas, l'alimentation de chacun des moyens élastiques se fait de préférence au moyen d'un rouleau d'alimentation rotatif réalisé par exemple en caoutchouc éventuellement strié, entraîné à vitesse variable et coopérant avec un galet de friction entrant en contact avec le rouleau de préférence sous l'action de son propre poids.

La superficie du tambour rotatif entraînant la feuille imperméable est de préférence maintenue à une température comprise entre 30 °C et 100 °C de façon à provoquer une prise immédiate de l'adhésif liquide porté par chaque moyen élastique dès que ledit moyen élastique entre en contact avec la feuille imperméable. Le maintien en température peut être obtenu soit par des moyens spécifiques pouvant comprendre une circulation de liquide de refroidissement soit simplement par l'utilisation d'un tambour de masse suffisante. Le choix de la température du tambour rotatif peut être aisément opéré par le technicien en tenant compte des paramètres que constituent la masse du tambour, la masse de l'adhésif par unité de longueur pour chacun des moyens élastiques, la température de l'adhésif au moment du contact ainsi que la nature de l'adhésif. Dans la pratique on constate qu'une différence de température d'au moins 20 °C est nécessaire et de préférence de 70 °C à 130 °C entre l'adhésif déposé à l'état liquide et la surface extérieure du tambour rotatif afin d'obtenir l'effet de collage quasiment immédiat désiré.

Dans l'installation permettant la mise en œuvre du procédé de l'invention, les moyens d'alimentation en continu pour les élastiques sont disposés le plus près possible du tambour rotatif et comprennent deux rouleaux exerçant un effet de pincement sur les moyens élastiques et entraînés à vitesse variable. Les moyens d'encollage sont montés en aval des moyens d'alimentation des élastiques, entre ces derniers et le tambour rotatif, et fonctionnent sans interruption. On comprend de cette manière que les moyens élastiques se trouvent encollés en continu alors que leur tension varie selon la commande d'un dispositif variateur des moyens d'alimentation.

La variation de vitesse des moyens d'alimentation peut être obtenue en utilisant tout dispositif approprié commandé en fonction de la position angulaire du tambour rotatif sur lequel se déroule la feuille imperméable.

On peut utiliser en particulier des rouleaux d'alimentation montés sur un arbre lequel est relié par deux trains d'engrenage possédant des rapports d'engrenage différents à un arbre d'entraînement par l'intermédiaire de deux embrayages commandés. Un capteur de position angulaire du tambour rotatif relié à un dispositif de commande fournit des signaux en vue de la commande deux embrayages. De cette manière il est possible d'obtenir des portions d'élastique non tendues et des portions d'élastique convenablement tendues, l'allongement pouvant aller, selon les élastiques utilisés, jusqu'à 300% ou même 400%.

Une autre possibilité consiste à utiliser des rouleaux d'alimentation montés sur un arbre entraîné par un moteur pas à pas. Ce moteur pas à pas est commandé par un disque codeur à lecture photoélectrique ou analogue, présentant sur son pourtour deux secteurs de traits différemment espacés et entraîné à vitesse constante.

La présente invention a également pour objet une couche-culotte à jeter obtenue par la mise en œuvre du procédé de l'invention. Cette couche-culotte à jeter comprend un coussin absorbant fixé à l'intérieur d'une enveloppe imperméable à l'humidité. L'enveloppe comporte des bords transversaux avant et arrière et une zone centrale d'entrejambes munie de découpes latérales pour permettre le passage des jambes. Des moyens élastiques sont fixés à l'enveloppe imperméable par collage au moins dans la zone de l'entrejambe. Les moyens élastiques comprennent de chaque côté du matelas absorbant, plusieurs brins individuels parallèles entre eux, chaque brin individuel comprenant une portion médiane encollée avec tension sur la zone centrale d'entrejambe et deux portions d'extrémité encollées sans tension sur les bords transversaux avant et arrière de l'enveloppe.

L'invention sera mieux comprise à l'étude de deux modes de réalisation particuliers décrits à titre d'exemples nullement limitatifs et illustrés par les dessins annexés sur lesquels:

la figure 1 est une vue en perspective schématique d'une partie d'une installation permettant la mise en œuvre du procédé de l'invention adapté à l'utilisation d'un ruban élastique;

la figure 2 montre de façon schématique un dispositif de commande pouvant être utilisé pour faire varier la tension des élastiques utilisés dans le procédé selon l'invention;

la figure 3 est une vue en perspective schématique d'une partie d'une variante d'installation permettant également la mise en œuvre du procédé de l'invention plus particulièrement adapté à l'utilisation d'une pluralité de brins élastiques individuels;

la figure 4 est une vue en perspective schématique selon la figure 3 montrant une variante de la commande de variation de la tension des élastiques; et

la figure 5 est une vue de dessus partiellement arrachée d'une couche-culotte selon l'invention à l'état étiré.

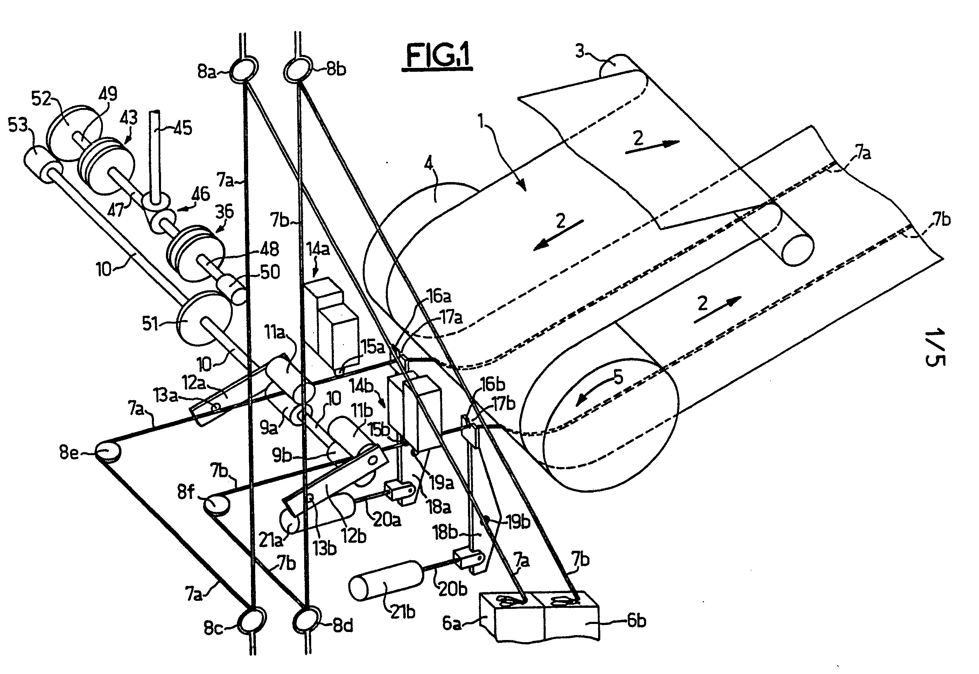

Comme on peut le voir sur la figure 1, une feuille mince imperméable 1 est déroulée en continu dans le sens des flèches 2 en passant sur un ensemble de rouleaux dont l'un 3 est visible sur la figure et un tambour rotatif 4 sur la surface périphérique duquel elle se trouve plaquée le long d'un trajet qui correspond sensiblement à la moitié de la circonférence du tambour 4. La feuille 1 mince et souple est réalisée en un matériau thermosoudable imperméable à l'humidité tel que du polyéthylène. Le tambour 4 est entraîné en rotation dans le sens de la flèche 5 et sa surface extérieure lisse est maintenue à une température relativement basse comprise entre 30 °C et 100 °C. Le maintien de cette température peut être obtenu par un refroidissement artificiel forcé à l'intérieur du tambour 4 ou simplement comme illustré sur la figure 1 grâce à l'utilisation d'un tambour métallique de masse suffisante.

Sur le côté de la machine se trouvent placés deux réceptacles 6a, 6b. A l'intérieur de chacun de ces réceptacles se trouve rangé en vrac un ruban élastique 7a, 7b par plis successifs de faible longueur superposés dans des plans également successifs, de sorte qu'en tirant sur l'extrémité libre supérieure du ruban 7a, 7b, il est possible d'extraire la totalité du ruban contenue dans le réceptacle 6a, 6b sans risquer la formation de noeuds ou d'amas qui gêneraient l'alimentation. Les rubans élastiques 7a, 7b issus de leur réceptacle 6a, 6b passent tout d'abord par deux premiers anneaux de guidage 8a, 8b disposés sensiblement au-dessus des réceptacles 6a, 6b et de l'ensemble de la machine. Les rubans élastiques 7a, 7b redescendent ensuite jusqu'à deux deuxième anneaux de guidage 8c, 8d. Le trajet des rubans élastiques 7a, 7b entre le réceptacle 6a, 6b d'une part et les deux anneaux de guidage respectifs 8a, 8b, 8c, 8d d'autre part permet de régulariser l'extraction des rubans élastiques sans exercer sur ces derniers de tension notable. Les boucles qui pourraient encore se former à la sortie des réceptacles 6a, 6b se trouvent éliminées au cours du trajet jusqu'aux premiers anneaux de guidage 8a, 8b ou par le passage à travers ces derniers.

Les deux rubans élastiques 7a, 7b sont dirigés à la sortie des anneaux 8c, 8d transversalement par rapport à l'axe longitudinal de la machine. Ils passent alors sur des galets 8e, 8f qui opèrent un changement de direction de 90° et à la sortie desquels les deux rubans élastiques 7a, 7b se trouvent parallèles à l'axe longitudinal de la machine et avec un écartement qui correspond à leur écartement final après encollage sur la feuille 1.

La machine étant symétrique par rapport à son axe longitudinal, on décrira maintenant l'ensemble des organes se trouvant d'un côté de l'axe qui seront affectés de la référence a. Les organes symétriques se trouvant de l'autre côté de l'axe sont identiques et visibles sur la fig. 1 affectés de la référence b.

Le ruban 7a passe entre un rouleau d'alimentation rotatif 9a solidaire d'un arbre 10 portant également le rouleau 9b et un galet de friction 11a pouvant tourner fou autour de son axe lequel est supporté par un levier 12a pouvant pivoter autour d'un axe fixe 13a. Le rouleau d'entraînement 9a peut comporter des stries non représentées sur la figure qui en augmentent la rugosité. Le galet de friction 11a est disposé au-dessus du rouleau d'alimentation 9a de façon que le galet de friction 11a vienne s'appuyer par son propre poids sur le rouleau 9a. Le ruban élastique 7a se trouve ainsi pincé entre le rouleau d'alimentation 9a et le galet de friction 11a. Dans ces conditions, le ruban élastique 7a possède, au moment de son passage entre le rouleau 9a et le galet 11a, une vitesse de déplacement linéaire correspondant à la vitesse de rotation de l'arbre 10 qui porte les rouleaux d'alimentation 9a, 9b.

Immédiatement en aval du rouleau d'alimentation 9a se trouve placé un dispositif d'encollage 14a comportant une buse 15a capable d'extruder un jet d'un adhésif liquide à haute température (hot melt) directement sur la face du ruban élastique 7a qui défile sous ladite buse 15a. La face du ruban adhésif 7a est maintenue horizontale comme on peut le voir sur la fig. 1 grâce à la disposition du rouleau d'alimentation 9a.

Le ruban élastique 7a ainsi encollé sur toute sa longueur en continu passe ensuite sur une patte de guidage 16a munie d'une gorge 17a et montée à l'extrémité d'un levier sensiblement vertical 18a qui peut pivoter autour d'un axe fixe 19a sous l'action de la tige 20a d'un vérin 21a. Un mouvement de pivotement des leviers 18a, 19b vers le tambour 4 permet d'écarter les rubans élastiques 7a, 7b des buses de distribution d'adhésif 15a, 15b lors des arrêts de la machine.

Le ruban élastique 7a subit une déviation légère vers le bas dans un plan vertical à l'issue de son passage dans la gorge de guidage 17a avant d'entrer en contact par sa face recouverte de liquide adhésif avec la face extérieure de la feuille mince 1 enroulée autour du tambour 4.

Sur la fig. 1 la distance entre les rouleaux d'alimentation 9a, 9b et le tambour rotatif 4 a été exagérée afin d'améliorer la clarté. Dans la réalité cette distance est réduite au minimum afin de limiter dans toute la mesure du possible l'amortissement des variations de tension dans le ruban élastique 7a, 7b dues aux variations de vitesse des rouleaux d'alimentation 9a, 9b.

Dès le contact avec la feuille 1, l'adhésif liquide porté par chacun des rubans élastiques 7a, 7b se trouve figé presque instantanément en raison de la différence de température entre l'adhésif liquide déposé à chaud par les buses 15a, 15b et la feuille 1 qui se trouve sensiblement à la même température que la périphérie du tambour rotatif 4 maintenu à une température relativement basse soit par une circulation interne de liquide réfrigérant, soit simplement par l'importance de sa masse métallique. Dans ces conditions, dès le contact avec la feuille 1 qui se déplace à grande vitesse dans le sens des flèches 2, les élastiques 7a, 7b se trouvent fixés par collage sur ladite feuille 1. Une différence de vitesse linéaire entre ladite feuille 1 et l'alimentation en rubans élastiques 7a, 7b par les deux rouleaux d'alimentation 9a, 9b provoque une tension des rubans élastiques 7a, 7b. Cette tension est obtenue par un ralentissement convenable de la vitesse de rotation des rouleaux d'alimentation 9a, 9b. Au contraire, lorsque la vitesse de rotation de ces rouleaux 9a, 9b est augmentée dans des proportions convenables de façon que la vitesse linéaire des rubans élastiques 7a, 7b à la sortie des dispositifs d'alimentation soit égale à la vitesse linéaire de la feuille 1 enroulée autour du tambour 4, les rubans élastiques 7a, 7b sont encollés sur la feuille 1 sans aucune tension.

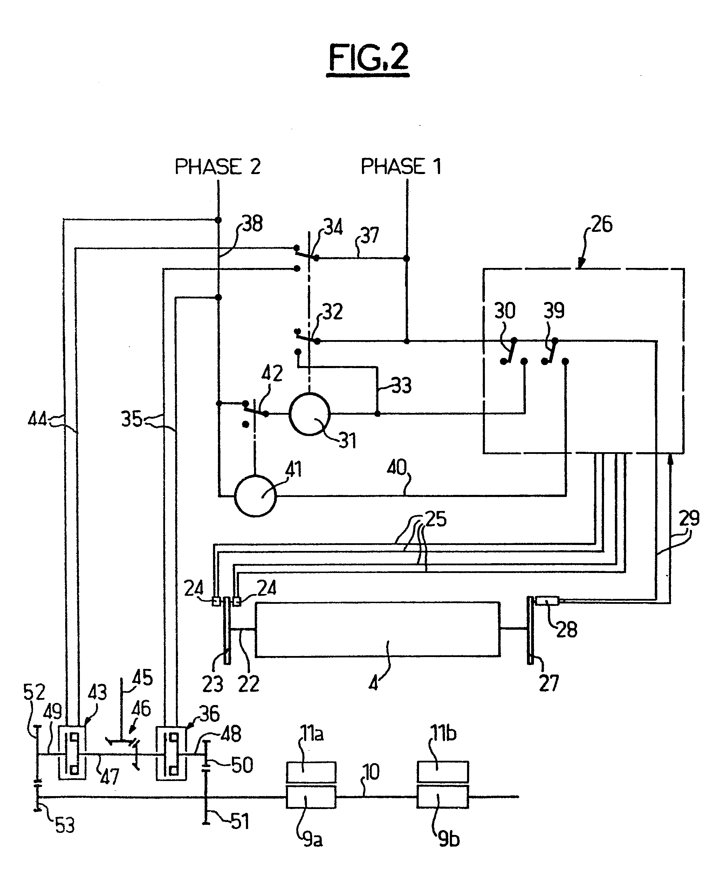

La fig. 2 sur laquelle les pièces identiques portent les mêmes références illustre un exemple de réalisation des moyens de variation de vitesse de rotation des rouleaux d'alimentation 9a, 9b portés par l'arbre 10.

On retrouve sur la fig. 2 le tambour rotatif 4 porté par son arbre 22 et comportant à l'une de ses extrémités un disque de codage 23 présentant une pluralité de marques dont le passage en face des capteurs 24 fournit chaque fois une impulsion qui est transmise par les connexions 25 à un dispositif compteur 26. A son autre extrémité, l'arbre 22 porte une deuxième roue de codage 27 comportant une seule marque coopérant avec un capteur capacitif 28 fournissant à chaque tour de l'arbre 22 un signal de remise à zéro transmis par les connexions 29 au dispositif compteur 26.

Le dispositif compteur 26 comporte des moyens non représentés pour mémoriser et sélectionner deux nombres par exemple compris entre 0 et 500 si le nombre de marques du disque de codage 23 est de 500.

Un premier interrupteur 30 est capable de se fermer et de s'ouvrir lorsque le dispositif compteur 26 est parvenu au premier nombre présélectionné. Lorsque l'interrupteur 30 est fermé, le circuit comportant le relais 31 est également fermé de sorte que ledit relais 31 est capable de fermer le contact 32 qui est ensuite maintenu fermé en raison de la connexion 33 et le contact 34 qui alimente en courant électrique par les connexions 35 l'organe de commande d'un premier embrayage 36 ou embrayage de petite vitesse. En effet lorsque le contact 34 est fermé, l'une des phases (phase 1) du courant d'alimentation est reliée par la connexion 37 tandis que l'autre phase (phase 2) est reliée par la connexion 38 aux connexions 35 de commande de l'embrayage 36.

Le dispositif de comptage 26 comprend un deuxième interrupteur 39 qui est capable de se fermer et de s'ouvrir pour le deuxième nombre présélectionné afin d'envoyer une impulsion de commande par la connexion 40 au relais 41 qui ouvre un instant le contact 42 normalement fermé. L'ouverture du contact 42 interrompt l'excitation du relais 31 et ouvre le circuit de maintien du contact 32. La même action provoque le passage du contact 34 de la position alimentant le premier embrayage 36 de petite vitesse vers la position représentée sur la fig. 2 où l'organe de commande du deuxième embrayage 43 dit de grande vitesse est alimenté par les connexions 44.

Un arbre d'entraînement à grande vitesse 45 transmet son couple de rotation par l'intermédiaire d'un train d'engrenages coniques 46 à un arbre intermédiaire 47 qui peut être relié alternativement aux deux demi-arbres 48 et 49 par l'intermédiaire des embrayages commandés 36 et 43. Le demi-arbre 48 coopérant avec l'embrayage 36 de petite vitesse prote un pignon 50 qui engrène avec une roue 51 solidaire de l'arbre 10.

Le demi-arbre 49 coopérant avec l'embrayage 43 de grande vitesse porte une roue 52 qui engrène avec un pignon 53 solidaire de l'arbre 10.

Dans ces conditions l'actionnement de l'un ou l'autre des embrayages 36, 43 modifie le rapport d'engrènement par lequel se fait la transmission du couple de rotation entre l'arbre d'entraînement 45 et l'arbre 10 qui porte les rouleaux d'alimentation 9a, 9b. On obtient ainsi deux vitesses de rotation différentes pour l'arbre 10.

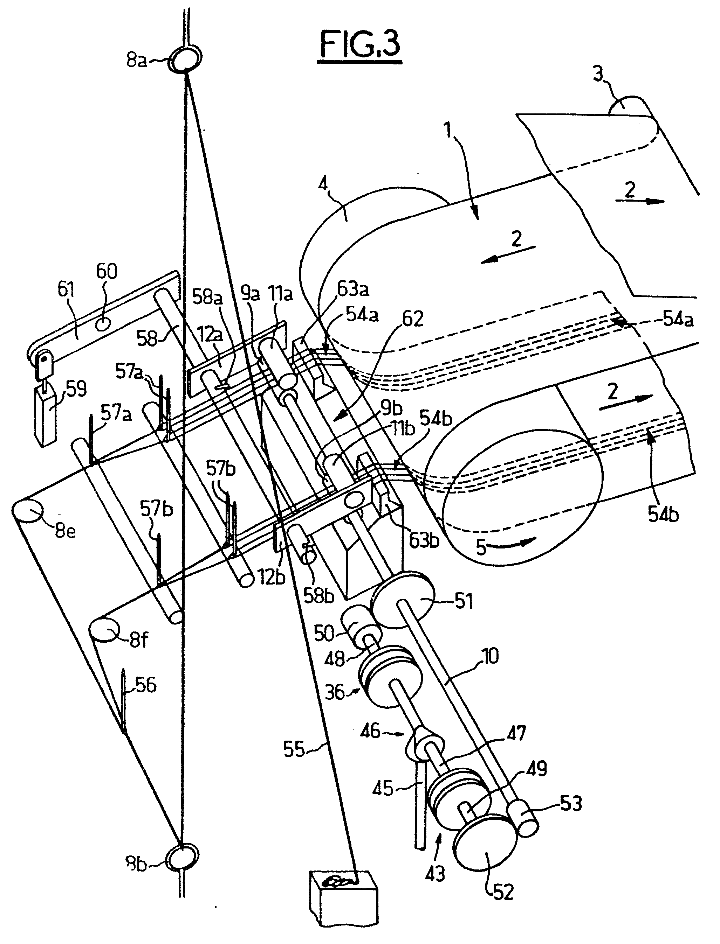

La fig. 3 sur laquelle les éléments identiques portent les mêmes références, illustre une variante de réalisation de l'invention qui est similaire dans son principe au mode de réalisation illustré sur la fig. 1 mais qui utilise, au lieu des deux rubans élastiques 7a, 7b, quatre brins individuels 54a, 54b qui sont obtenus à partir d'un ruban multibrins unique 55 subissant une succession de séparations par passage sur des tiges de séparation 56, 57a, 57b disposées sur son trajet. On retrouve également dans ce mode de réalisation les rouleaux d'alimentation 9a, 9b entraînés avec une vitesse variable par l'arbre 10. Les leviers 12a, 12b des galets presseurs 11a, 11b sont ici montés libres en rotation sur une tige transversale 58 qui peut subir un mouvement vers le haut sous l'action d'un vérin de commande 59 entraînant le pivotement autour de son axe fixe 60 d'un levier 61. Des butées 58a, 58b solidaires de la tige 58 limitent le mouvement de pivotement vers le bas des leviers 12a, 12b. De cette manière, le mouvement vers le haut de la tige 58 soulève les leviers 12a, 12b et les galets 11a, 11b dès que les leviers 12a, 12b entrent en contact avec les butées 58a, 58b. La poursuite de ce mouvement vers le haut de la tige 58 soulève les brins individuels 54a, 54b de leur bloc d'encollage 63a, 63b lors des arrêts de la machine.

Dans ce mode de réalisation, l'encollage se fait par passage des brins individuels 54a, 54b sur deux blocs d'encollage 63a, 63b qui comportent des gorges adaptées au passage de chacun des brins individuels, lesdites gorges étant remplies d'adhésif liquide à haute température (hot melt) de façon à enrober complètement chaque brin individuel lors de son passage dans ladite gorge. Pour plus de précision sur ce mode d'encollage et sur l'utilisation d'élastiques multibrins on pourra se reporter à la demande de brevet n° 83 00 780 déposée le 19 janvier 1983 au nom de la demanderesse.

Grâce à la disposition visible sur la fig. 3 des rouleaux d'alimentation 9a, 9b entraînés en rotation à vitesse variable et disposés immédiatement en amont des blocs d'encollage 63a, 63b on obtient le même résultat qu'avec le mode de réalisation de la fig. 1 c'est-à-dire une tension variable des brins individuels élastiques 54a, 54b qui sont par ailleurs encollés en continu.

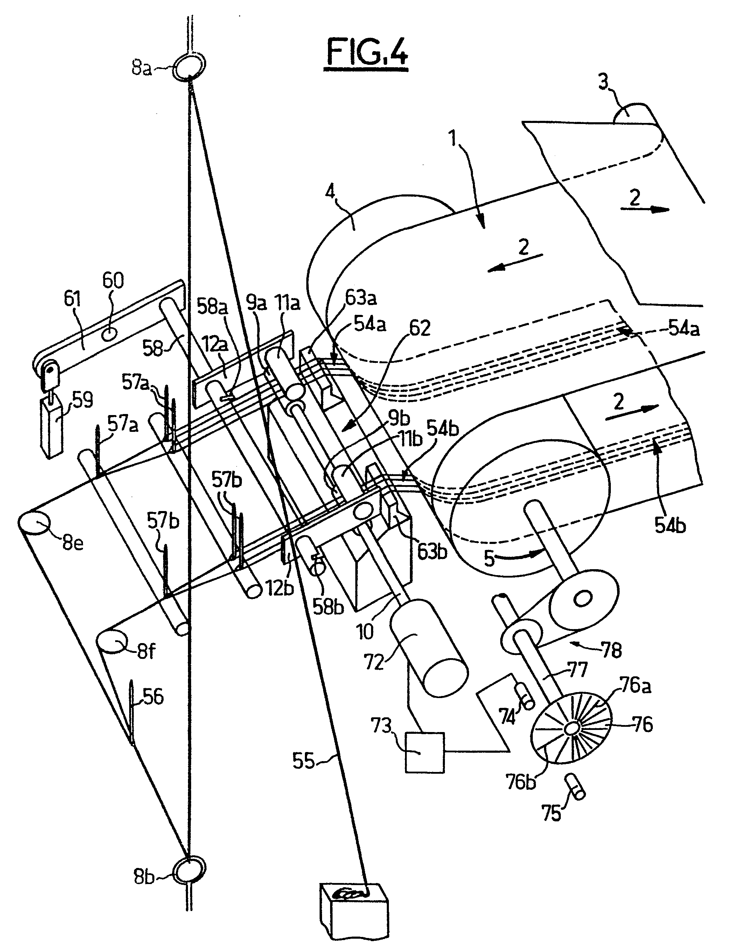

Sur la figure 4 qui illustre une variante de la commande de variation de la tension des élastiques, on retrouve tous les éléments de la figure 3, sauf les moyens de commande de l'arbre 10 portant les rouleaux d'alimentation 9a, 9b.

L'arbre 10 est ici entraîné par un moteur électrique pas à pas 72 alimenté par un circuit de commande 73. Le circuit de commande reçoit les impulsions d'un récepteur photo-électrique 74 éclairé par un émetteur 75 à travers un disque codeur 76 présentant une alternance de traits transparents et opaques. Le disque codeur 76 comporte sur son pourtour, deux secteurs 76a, 76b présentant l'un (76a) des traits transparents plus rapprochés et l'autre (76b) des traits transparents plus espacés. Le disque codeur 76 est monté sur un arbre 77 qui est accouplé, par exemple comme représenté par une transmission à chaîne 78, avec le tambour 4 de manière à effectuer un tour complet lorsque le tambour effectue une rotation provoquant le passage d'une longueur de feuille 1 correspondant à une couche-culotte. Par ailleurs, le nombre de traits transparents du disque codeur 76 et le moteur pas à pas 72 sont choisis de manière que le moteur 72 effectue un tour complet à chaque tour du disque 76. Par conséquent, les traits différemment espacés du disque codeur 76 entraîné à vitesse constante produisent une variation périodique de la vitesse du moteur pas à pas 72 et des rouleaux d'alimentation 9a, 9b, donc de la tension des brins élastiques 54a, 54b.

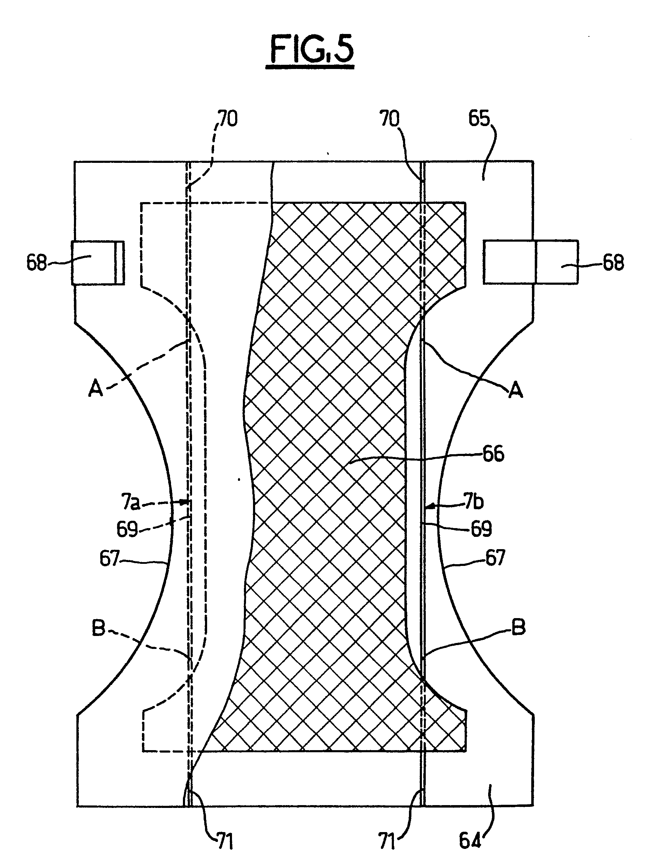

La figure 5 montre une couche-culotte obtenue selon le procédé de l'invention et représentée, pour simplifier le dessin, en position étirée. On retrouve la feuille imperméable 1 et les rubans élastiques 7a, 7b. L'enveloppe extérieure imperméable constitue par la feuille 1 comporte un bord transversal avant 64 et un bord transversal arrière 65. Un matelas absorbant 66 est fixé sur la face interne de la feuille 1. Deux découpes latérales arrondies 67 permettent de faciliter le passage des jambes. Enfin, deux attaches adhésives 68 sont fixées sur le bord arrière 65 et permettent la fermeture de la couche-culotte.

Comme on peut le voir sur la figure 5, les deux rubans élastiques 7a, 7b sont fixés sur toute leur longueur de façon rectiligne parallèlement à l'axe longitudinal de la couche-culotte. Ils comportent une portion médiane 69 encollée avec tension sur la zone centrale d'entrejambe entre les points A et B et deux portions d'extrémité 70 et 71 également encollées mais cette fois sans tension sur les bords transversaux 64 et 65.

On notera que les rubans élastiques 7a, 7b se trouvent fixés immédiatement au voisinage des bords du matelas absorbant 66 dans la zone de l'entrejambe.

Bien que la description ait été faite en relation avec l'utilisation d'élastiques disposés de façon rectiligne on comprendra que l'invention pourrait également s'appliquer sans modification notable à la pose d'élastiques curvilignes encollés sur toute leur longueur et comportant des portions de tension variable. Il suffirait en effet de prévoir des moyens d'entraînement en translation transversale cyclique de l'ensemble des moyens d'alimentation et d'encollage coopérant avec chaque élastique sans modifier le fonctionnement de ces organes tel qu'il a été décrit. Le point de contact de chaque élastique avec la feuille imperméable serait alors déplacé périodiquement ce qui entraînerait le collage d'un élastique selon une trajectoire curviligne pouvant par exemple suivre le profil des découpes latérales arrondies de l'entrejambe.

Par ailleurs, bien que dans les exemples illustrés la vitesse d'alimentation des élastiques ait comporté deux valeurs déterminées, on comprendra que l'invention ne soit pas limitée à l'utilisation de deux valeurs. Au contraire, on pourrait sans modification importante utiliser plus de deux valeurs ou même une variation continue afin d'obtenir des zones de tension variable pour les élastiques encollés, permettant par exemple d'adapter la tension de chaque portion d'élastique aux caractéristiques de confort et d'étanchéité requises pour chaque portion de la couche-culotte.

Enfin, il va de soi que la commande de variation de la tension des moyens élastiques, au lieu d'être dérivée du mouvement de rotation du tambour 4 sur lequel passe la feuille 1, pourrait être dérivée de tout autre organe rotatif effectuant un tour par article (couche-culotte).

## Revendications

1. Procédé de fabrication en continu de couches-culottes à jeter comprenant un coussin absorbant fixé à l'intérieur d'une enveloppe imperméable à l'humidité comportant des bords transversaux avant et arrière et une zone centrale d'entrejambe munie de découpes latérales pour permettre le passage des jambes et de moyens élastiques fixés à l'enveloppe imperméable par collage au moins dans la zone de l'entrejambe et mise en contact avec la face interne de l'enveloppe imperméable entraînée en continu par un tambour rotatif (4) à surface lisse, dans lequel lesdits moyens élastiques alimentés en continu sont encollés en continu sur toute leur longueur par application d'un adhésif liquide à haute température et où l'on fait varier périodiquement la tension exercée sur lesdits moyens élastiques aux points de contacts avec l'enveloppe imperméable (1) depuis une valeur proche de zéro correspondant aux portions des moyens élastiques encollées sur les bords transversaux avant et arrière, jusqu'à une valeur maximale correspondant aux portions des moyens élastiques encollées sur la zone centrale d'entrejambe, chaque moyen élastique passant entre deux organes d'alimentation rotatifs capables d'exercer un effet de pincement sur les moyens élastiques et entraînés à vitesse variable pour provoquer la tension variable des moyens élastiques en aval des organes d'alimentation et l'encollage des moyens élastiques étant réalisé en aval des organes d'alimentation précités.

2. Procédé selon la revendication 1, caractérisé par le fait que chacun des moyens élastiques est constitué par un ruban élastique (7a, 7b) recevant sur l'une de ses faces un adhésif liquide à haute température déposé en continu.

3. Procédé selon la revendication 1, caractérisé par le fait que chacun des moyens élastiques est constitué par une pluralité de brins élastiques individuels (54a, 54b) obtenus par séparation en continu à partir d'un ruban élastique multibrins (55), chaque brin individuel étant enrobé en continu sur toute sa périphérie par un adhésif liquide à haute température.

4. Procédé selon l'une quelconque des revendications précédentes, caractérisé par le fait que l'alimentation de chaque moyen élastique se fait par un rouleau d'alimentation rotatif (9a, 9b) entraîné à vitesse variable coopérant avec un galet de friction (11a, 11b) entrant en contact avec le rouleau d'alimentation sous l'action de son propre poids.

5. Procédé selon l'une quelconque des revendications précédentes, caractérisé par le fait que la superficie du tambour rotatif (4) est maintenue à une température comprise entre 30 °C et 100 °C de façon à provoquer une prise immédiate de l'adhésif liquide porté par chaque moyen élastique.

6. Installation de fabrication en continu de couches-culottes à jeter comprenant un coussin absorbant fixé à l'intérieur d'une enveloppe imperméable à l'humidité et des moyens élastiques fixés à l'enveloppe imperméable par collage, l'installation comprenant des moyens d'alimentation en continu d'une feuille de matière plastique mince destinée à constituer l'enveloppe imperméable, incluant un tambour rotatif à surface lisse autour duquel est partiellement enroulée ladite feuille, des moyens d'alimentation en continu de moyens élastiques et des moyens d'encollage en continu des élastiques par application d'un adhésif liquide à haute température, caractérisé par le fait que les moyens d'alimentation des élastiques sont disposés le plus près possible du tambour et comprennent deux rouleaux exerçant un effet de pincement sur les moyens élastiques et entraînés à vitesse variable et que les moyens d'encollage sont montés en aval des moyens d'alimentation, entre ces derniers et le tambour rotatif.

7. Installation selon la revendication 6, caractérisé par le fait qu'un dispositif de guidage (17a, 17b) est placé entre les moyens d'encollage (14a, 14b) et le tambour (4) pour dévier la trajectoire des moyens élastiques (7a, 7b) en direction du tambour.

8. Installation selon les revendications 6 ou 7, caractérisée par le fait que les moyens d'alimentation comprennent un rouleau d'alimentation rotatif (9a, 9b) entraîné à vitesse variable coopérant avec un galet de friction (11a, 11b) entrant en contact sous l'action de son propre poids avec ledit rouleau d'alimentation.

9. Installation selon la revendication 8, caractérisé par le fait que l'arbre (10) portant les rouleaux d'alimentation (9a, 9b) est relié par deux trains d'engrenages (50, 51; 52, 53) possédant des rapports d'engrenages différents à un arbre d'entraînement (45) par l'intermédiaire de deux embrayages commandés (36, 43).

10. Installation selon la revendication 9, caractérisée par le fait qu'elle comprend un capteur de position angulaire (24) du tambour rotatif (4) relié à un dispositif de commande (26) fournissant des signaux en vue de la commande des deux embrayages (36, 43).

11. Installation selon la revendication 8, caractérisée par le fait que l'arbre (10) portant les rouleaux d'alimentation (9a, 9b) est entraîné par un moteur pas à pas (72) commandé par un disque codeur (76) à lecteur photo-électrique ou analogue, présentant sur son pourtour deux secteurs (76a, 76b) de traits différemment espacés et entraîné à vitesse constante.

12. Couche-culotte à jeter comprenant un coussin absorbant (66) fixé à l'intérieur d'une enveloppe imperméable à l'humidité (1) comportant des bords transversaux avant et arrière (64, 65) et une zone centrale d'entrejambe munie de découpes latérales (67) pour permettre le passage des jambes et de moyens élastiques fixés à l'enveloppe imperméable par collage au moins dans la zone de l'entrejambe, caractérisée par le fait qu'elle a été fabriquée selon le procédé de l'une des revendications 1 à 5 et que les moyens élastiques comprennent de chaque côté du matelas absorbant, plusieurs brins individuels parallèles entre eux, chaque brin individuel comprenant une portion médiane encollée avec tension sur la zone centrale d'entrejambe et deux portions d'extrémité encollées sans tension sur les bords transversaux avant et arrière de l'enveloppe.

**Patentansprüche**

1. Verfahren zur kontinuierlichen Herstellung von Wegwerfwindeln mit einem im Inneren einer feuchtigkeitsundurchlässigen Hülle mit vorderen und hinteren Querrändern und einer mittigen Zwickelzone mit seitlichen Ausschnitten zum Durchlassen der Beine und an der undurchlässigen Hülle mindestens in der Zwickelzone festgeklebten und mit der Innenfläche der kontinuierlich durch eine Drehtrommel (4) mit glatter Oberfläche mitgenommenen undurchlässigen Hülle in Berührung gebrachten elastischen Mitteln befestigen Saugkissen, wobei die kontinuierlich zugeführten elastischen Mittel kontinuierlich auf ihrer gesamten Länge durch Auftragen eines bei hoher Temperatur flüssigen Klebstoffs gummiert werden und periodisch die auf die elastischen Mittel an den Berührungspunkten mit der undurchlässigen Hülle (1) ausgeübte Spannung von einem Wert nahe Null, entsprechend den an den vorderen und hinteren Querrändern gummierten Bereichen der elastischen Mittel, bis zu einem Maximalwert, entsprechend den auf der Mittigen Zwickelzone gummierten Bereichen der elastischen Mittel, verändert wird, wobei jedes elastische Mittel durch zwei Zufuhrdrehorgane durchläuft, welche zum Ausüben eines Quetscheffekts auf die elastischen Mittel geeignet sind und mit variabler Geschwindigkeit angetrieben werden, um die variable Spannung der elastischen Mittel stromabwärts der Zufuhr- und Klebeorgane für die stromabwärts dieser Zufuhrorgane gefertigten elastischen Mittel zu bewirken.

2. Verfahren nach Anspruch 1, dadurch gekennzeichnet, dass jedes elastische Mittel aus einem elastischen Band (7a, 7b) besteht, auf welches auf eine Seite ein bei hoher Temperatur flüssiger Klebstoff kontinuierlich aufgebracht wird.

3. Verfahren nach Anspruch 1, dadurch gekennzeichnet, dass jedes elastische Mittel aus einer Mehrzahl von einzelnen elastischen Litzen (54a, 54b) besteht, welche durch kontinuierliche Trennung aus einem viellitzigen elastischen Band (55) hergestellt werden, wobei jede einzelne Litze kontinuierlich auf ihrer gesamten Umfangsfläche mit einem bei hoher Temperatur flüssigen Klebstoff umgeben wird.

4. Verfahren nach einem der vorhergehenden Ansprüche, dadurch gekennzeichnet, dass die Zufuhr eines jeden elastischen Mittels durch eine mit variabler Geschwindigkeit angetriebene Zufuhrdrehrolle (9a, 9b), welche mit einer unter der Wirkung ihres eigenen Gewichts mit der Zufuhrrolle in Berührung kommenden Reibrolle (11a, 11b) zusammenwirkt, erfolgt.

5. Verfahren nach einem der vorhergehenden Ansprüche, dadurch gekennzeichnet, dass die Oberfläche der Drehtrommel (4) bei einer Temperatur zwischen 30 °C und 100 °C gehalten wird, so dass ein unmittelbares Abbinden des von jedem elastischen Mittel getragenen flüssigen Klebstoffs bewirkt wird.

6. Einrichtung zur kontinuierlichen Herstellung von Wegwerfwindeln mit einem in Inneren einer feuchtigkeitsundurchlässigen Hülle befestigten Saugkissen und an der undurchlässigen Hülle festgeklebten elastischen Mitteln, wobei die Vorrichtung Mittel zur kontinuierlichen Zufuhr einer dünnen Folie aus Plastikmaterial zur Bildung der undurchlässigen Hülle mit einer Drehtrommel mit glatter Oberfläche, um welche die Folie teilweise aufgerollt wird, Mittel zur kontinuierlichen Zufuhr der elastischen Mittel und Mittel zum kontinuierlichen Gummieren der elastischen Mittel durch Auftragen eines bei hoher Temperatur flüssigen Klebstoffs umfasst, dadurch gekennzeichnet, dass die Mittel zur Zufuhr der elastischen Mittel so nahe wie möglich an der Trommel angeordnet sind und zwei Rollen umfassen, welche einen Quetscheffekt auf die elastischen Mittel ausüben und mit variabler Geschwindigkeit angetrieben werden und dass die Mittel zum Gummieren stromabwärts der Zufuhrmittel zwischen letzteren und der Drehtrommel angeordnet sind.

7. Einrichtung nach Anspruch 6, dadurch gekennzeichnet, dass eine Führungsvorrichtung (17a, 17b) zwischen den Gummiermitteln (14a, 14b) und der Trommel (4) angeordnet ist, um die Bahn der elastischen Mittel (7a, 7b) in Richtung der Trommel abzulenken.

8. Einrichtung nach Anspruch 6 oder 7, dadurch gekennzeichnet, dass die Zufuhrmittel eine mit variabler Geschwindigkeit angetriebene Zufuhrdrehrolle (9a, 9b), welche mit einer unter der Wirkung ihres eigenen Gewichts mit der Zufuhrrolle in Berührung kommenden Reibrolle (11a, 11b) zusammenwirkt, umfassen.

9. Einrichtung nach Anspruch 8, dadurch gekennzeichnet, dass die die Zufuhrrollen (9a, 9b) tragende Welle (10) durch zwei Getriebezüge (50, 51; 52, 53) mit verschiedenem Übersetzungsverhältnis mittels zweier gesteuerter Kupplungen (36, 43) mit einer Getriebewelle (45) verbunden ist.

10. Einrichtung nach Anspruch 9, dadurch gekennzeichnet, dass sie einen mit einer Signale zur Steuerung der zwei Kupplungen (36, 43) liefernden Steuervorrichtung (26) verbundenen Winkellagegeber (24) der Trommel (4) umfasst.

11. Einrichtung nach Anspruch 8, dadurch gekennzeichnet, dass die die Zufuhrrollen (9a, 9b) tragende Welle (10) durch einen durch eine Codierscheibe (76) mit photoelektrischem oder analogem Leser, welche auf ihrem Umfang zwei Sektoren (76a, 76b) mit Strichen mit verschiedenem Abstand aufweist und mit konstanter Geschwindigkeit angetrieben wird, gesteuerten Schrittmotor angetrieben wird.

12. Wegwerfwindel mit einem im Inneren einer feuchtigkeitsundurchlässigen Hülle (1) mit vorderen und hinteren Querrändern (64, 65) und einer mit seitlichen Ausschnitten (67) zum Durchlassen der Beine vorgesehenen mittigen Zwickelzone und an der undurchlässigen Hülle wenigstens in der Zwickelzone festgeklebten elastischen Mitteln befestigten Saugkissen, dadurch gekennzeichnet, dass sie nach dem Verfahren nach einem der Ansprüche 1 bis 5 hergestellt ist und dass die elastischen Mittel auf beiden Seiten saugfähiges Material und mehrere einzelne zueinander parallele Litzen aufweisen, wobei jede einzelne Litze einen mit Spannung auf die mittige Zwickelzone aufgeklebten Mittelbereich und zwei ohne Spannung auf die vorderen und hinteren Querränder der Hülle geklebte Endbereiche aufweist.

**Claims**

1. Process for the continuous manufacture of disposable integral diapers comprising an absorbent pad secured inside a moisture-impervious enclosure comprising front and rear transverse edges and a central crotch region which is provided with side cutouts to allow legs to pass through and elastic means secured to the impervious enclosure by adhesion at least in the crotch region and by placing in contact with the inner face of the impervious enclosure which is driven continuously by a smooth-surfaced rotary drum (4), in which the said elastic means which are supplied continuously are continuously coated with adhesive over their entire length by application of a hot-melt adhesive and where the tension applied to the said elastic means at points of contact with the impervious enclosure (1) is varied periodically from a value close to zero, corresponding to the portions of the elastic means which are adhered to the front and rear transverse edges, to a maximum value corresponding to the portions of the elastic means which are adhered to the central crotch region, each elastic means passing between two rotary supply devices capable of exerting a pinching effect on the elastic mean and drived at variable speed to produce the variable tension in the elastic means downstream of the supply devices and the coating of the elastic means with adhesive being performed downstream of the abovementioned supply devices.

2. Process according to Claim 1, characterized in that each of the elastic means consists of an elastic strip (7a, 7b) receiving a hot-melt adhesive deposited continuously on one of its faces.

3. Process according to Claim 1, characterized in that each of the elastic means consists of a plurality of individual elastic strands (54a, 54b) produced by continuous separation from an elastic multistrand tape (55), each individual strand being continuously coated with a hot-melt adhesive over its entire periphery.

4. Process according to any one of the preceding claims, characterized in that the supply of each elastic means is effected by a rotary supply roll (9a, 9b) driven at a variable speed and interacting with a friction roller (11a, 11b) coming into contact with the supply roll under the effect of its own weight.

5. Process according to any one of the preceding claims, characterized in that the surface of the rotary drum (4) is maintained at a temperature of between 30 °C and 100 °C so as to cause an immediate setting of the hot-melt carried by each elastic means.

6. Equipment for the continuous manufacturing of disposable integral diapers comprising an absorbent pad secured inside a moisture-impervious enclosure and elastic means secured by adhesion to the impervious enclosure, the equipment comprising means for continuously supplying a sheet of thin plastic intended to form the impervious enclosure, including a smooth-surfaced rotary drum around which the said sheet is partly wound, means for continuously supplying elastic means and means for continuously coating the elastic means with adhesive by the application of a hot-melt adhesive characterized in that the means for supplying the elastic means are arranged as close as possible to the drum and comprise two rolls exerting a pinching action on the elastic means and driven at a variable speed and in that the means for coating with adhesive are mounted downstream of the supply means between the latter and the rotary drum.

7. Equipment according to Claim 6, characterized in that a guiding device (17a, 17b) is placed between the means for coating with adhesive (14a, 14b) and the drum (4) in order to divert the path of the elastic means (7a, 7b) towards the drum.

8. Equipment according to Claims 6 or 7, characterized in that the supply means comprises a rotary supply roll (9a, 9b) driven at a variable speed and interacting with a friction roller (11a, 11b) coming into contact with the said supply roll under the effect of its own weight.

9. Equipment according to Claim 8, characterized in that the shaft (10) carrying the supply rolls (9a, 9b) is coupled by means of two sets of gears (50, 51; 52, 53) having different gear ratios to a driving shaft (45) by means of two controlled clutches (36, 43).

10. Equipment according to Claim 9, characterized in that it comprises a sensor of the angular position (24) of the rotary drum (4) linked to a control device (26) supplying signals for control of the two clutches (36, 43).

11. Equipment according to Claim 8, characterized in that the shaft (10) carrying the supply rolls (9a, 9b) is driven by a stepping motor (72) controlled by a coding disc (76) with a photoelectric or similar reader, having on its periphery two sectors (76a, 76b) with differently spaced lines and driven at a constant speed.

12. Disposable integral diaper comprising an absorbent pad (66) secured inside a moisture-impervious enclosure (1) comprising front and rear transverse edges (64, 65) and a central crotch region which is provided with side cutouts (67) to allow legs to pass through and elastic means secured to the impervious enclosure by adhesion at least in the crotch region, characterized in that it has been manufactured according to the process of one of Claims 1 to 5 and in that the elastic means comprise, on each side of the absorbent pad, several individual strands which are parallel to each other, each individual strand comprising a median portion adhered under tension on the central crotch region and two end portions adhered without tension on the front and rear transverse edges of the enclosure.

1/5

FIG.1

# FIG.2

# FIG.3

# FIG.4

# FIG.5